# EUROPEAN PATENT SPECIFICATION

(11) **EP 2 825 221 B1**
(45) Date of publication and mention of the grant of the patent: **21.02.2018**
(21) Application number: 13761200.8
(22) Date of filing: 11.03.2013
(51) Int. Cl.: A61M 1/16

(54) **APPARATUS AND METHOD FOR VENTING GAS FROM A LIQUID**
VORRICHTUNG UND VERFAHREN ZUM ABLENKEN VON GAS AUS EINER FLÜSSIGKEIT
APPAREIL ET PROCÉDÉ DE DÉGAZAGE D'UN LIQUIDE

(30) Priority: 12.03.2012 US 201261609587 P
(43) Date of publication of application: 21.01.2015
(73) Proprietor: Fresenius Medical Care Holdings, Inc., Waltham, MA 02451 (US)
(72) Inventor: CRNKOVICH, Martin Joseph, Walnut Creek, CA 94598 (US); WEAVER, Colin, Pleasanton, CA 94566 (US)
(74) Representative: Kirkham, Nicholas Andrew
(86) International application number: PCT/US2013/030182
(87) International publication number: WO 2013/138233

(56) References cited:
- EP-A1- 0 900 098
- WO-A1-98/39080
- WO-A1-2010/142394
- US-A- 4 828 543
- US-A1- 2009 071 911
- US-A1- 2009 152 179
- US-A1- 2009 320 684
- US-A1- 2010 030 151

## Description

### RELATED APPLICATION

This application claims the benefit of U.S. Provisional Application No. 61/609,587, filed on March 12, 2012. The entire teachings of U.S. Provisional Application No. 61/609,587, filed on March 12, 2012 and U.S. Provisional Application No. 61/470,680, filed on April 1, 2011, are incorporated herein by reference.

### BACKGROUND OF THE INVENTION

Hemodialysis is the diffusive transfer of small solutes out of blood plasma by diffusion across a selectively permeable membrane. Hemodialysis proceeds due to a concentration gradient across the selectively permeable membrane such that solutes diffuse from a liquid having a higher concentration to a liquid having a lower concentration. Hemodialysis removes toxic substances, metabolic waste, and excess fluid from the bloodstream using an extracorporeal circuit with components designed to perform ultrafiltration and diffusion on the blood. Before the blood is returned to the body, air bubbles are removed from the blood to inhibit embolisms.

Gas venting chambers for hemodialysis systems have been disclosed in the art. For example, a conventional system is disclosed in U.S. Patent No. 7,871,391, which describes a chamber for use in an extracorporeal liquid system. The conventional system includes a semipermeable membrane at the top of the gas vent chamber that allows gas in the liquid to vent from the chamber. In such a system, it is important to minimize contact between the liquid (e.g., blood) and the semipermeable membrane. Should the blood contact the membrane, proteins present within the blood can be deposited on the membrane, thus clogging the membrane and decreasing the ability of gas (e.g., air) to exit through the membrane. A gas venting system without gas-source to control the pressure differential over a semipermeable membrane is known from document US2010030151 in a medical liquid delivery system.

Despite the fact that there are existing systems for venting a gas from a liquid, there is a need for improved systems that are reliable, affordable, and simple to use in either a clinical setting or in the home. In particular, there is a need for an apparatus and method for venting gas from a liquid that prevents the liquid in the gas collection chamber from contacting a semipermeable membrane covering the outlet where the gas vents to the atmosphere.

In addition, normal operation of a hemodialysis machine results in gas build-up in a gas collection chamber that is vented through a semipermeable membrane. The continual release of gas through the semipermeable membrane can tend to degrade the membrane due to the pressure applied across the membrane by the gas. Thus there is also a need for an apparatus and method for prolonging the lifetime of the semipermeable membrane.

### SUMMARY OF THE INVENTION

Aspects of the present invention relate to a gas venting apparatus and a method for venting gas, which are applicable to a wide variety of medical liquid delivery systems. The embodiments discussed below, however, are directed generally to dialysis, such as hemodialysis ("HD").

In one embodiment of this invention, an apparatus for venting gas contained in a liquid flowing in a liquid flow circuit includes a gas collection chamber located within the liquid flow circuit so that liquid flows through the chamber allowing gas to separate from the liquid and establish a gas-liquid interface within the chamber. A gas vent chamber is provided at the top of the gas collection chamber through which gas within the chamber can be released. The gas collection chamber and the gas vent chamber can be a single unit. A lower detector located at either the gas collection chamber or the gas vent chamber, and an upper detector located at either the gas collection chamber or the gas vent chamber are provided. The lower detector is located below the upper detector. The lower and upper detectors are capable of detecting gas and liquid. A clamp is provided in the gas vent chamber either between the lower and the upper level detectors or above both level detectors. The apparatus also includes a control apparatus for opening and closing the clamp in response to whether the lower and upper detectors detect gas or liquid.

In another embodiment of this invention, an automated method for venting a gas contained in a liquid flowing in a liquid flow circuit includes flowing liquid into a gas collection chamber located within the liquid flow circuit so that liquid flows through the gas collection chamber allowing gas to separate from the liquid and establish a gas-liquid interface within the gas collection chamber, detecting whether liquid is present at a lower position in either the gas collection chamber or a gas vent chamber by a lower level detector for detecting gas and liquid, opening a clamp if a liquid is not present at the lower position, detecting whether liquid is present at an upper position in either the gas collection chamber or the gas vent chamber by an upper level detector for detecting gas and liquid, and closing the clamp if liquid is present at the upper position. The clamp can be located in the gas vent chamber either between the lower level detector and the upper level detector or above both level detectors.

In another embodiment of this invention, an apparatus for controlling pressure differential across a semipermeable membrane sealing an exit pathway from a gas venting chamber is disclosed. The apparatus includes a first pressure sensor that senses the pressure along an exit pathway from a gas venting chamber, a valve operably connected to a gas source, the exit pathway from the gas collection chamber, and the atmosphere, and a control apparatus connected to the first pressure sensor and the valve. The control apparatus can vary the gas source in order to vary the amount of backpressure applied across the semipermeable membrane. A gas pump or pressurized gas, such as pressurized air or nitrogen, can provide the gas source. The apparatus can further include a second pressure sensor that senses the inlet or outlet pressure. The control apparatus can compare the pressure measured by the first pressure sensor and the second pressure sensor and control the gas source so that the amount of pressure along the exit pathway is less than the pressure measured by the second pressure sensor. The pressure sensed by the second pressure sensor can be approximately 100 mmHg to approximately 500 mmHg, or approximately 100 mmHg to approximately 200 mmHg. The apparatus can further include a lower level detector located in either the gas collection chamber or the gas vent chamber, and an upper level detector located in either the gas collection chamber or the gas vent chamber. The lower level detector can be located below the upper level detector, and the lower and upper level detectors can be capable of detecting gas or liquid. The control apparatus can open the valve connection to the atmosphere when the lower level detector detects gas and close the valve connection to the atmosphere when the upper level detector detects liquid.

In another embodiment, an apparatus for controlling the pressure differential across a semipermeable membrane sealing an exit pathway from a gas venting chamber that receives liquid-releasing gas is disclosed. The apparatus includes a gas source for applying pressure to the exit pathway side of the semipermeable membrane, a valve operably connected to the gas source, the exit pathway, and the atmosphere, a first pressure sensor that senses the pressure along the exit pathway side, a second pressure sensor that detects the pressure in the gas venting chanber, and a control means connected to the first and second pressure sensors and the valve. The control means can vary the gas source in order to vary the amount of backpressure applied across the semipermeable membrane.

In another embodiment, an automated method for controlling pressure differential across a semipermeable membrane includes detecting an inlet or outlet pressure, detecting a pressure along an exit pathway, comparing the inlet or outlet pressure to the exit pathway pressure with a control apparatus, increasing the amount of pressure supplied by a gas source if the inlet pressure exceeds the exit pathway pressure by a first predetermined amount, and decreasing the amount of pressure supplied by the gas source if the inlet pressure is too close to the exit pathway pressure by a second predetermined amount.

In another embodiment, an automated method for controlling pressure differential across a semipermeable membrane includes detecting an inlet or outlet pressure, detecting a pressure along an exit pathway, comparing the inlet or outlet pressure to the exit pathway pressure with a control apparatus, opening a valve connection to the atmosphere if the inlet pressure exceeds the exit pathway pressure by a first predetermined amount, and closing the valve connection to the atmosphere if the inlet pressure is too close to the exit pathway pressure by a second predetermined amount.

In another embodiment, an automated method for controlling pressure differential across a semipermeable membrane includes detecting whether liquid is present at a lower level detector, opening a valve connection to the atmosphere if liquid is not present at the lower level detector, detecting whether liquid is present at an upper level detector, and closing the valve connection to the atmosphere if liquid is present at the upper level detector.

In another embodiment of this invention, an extracorporeal hemodialysis circuit includes arterial tubing for receiving unfiltered blood from a patient, venous tubing for providing filtered blood to a patient, a dialyzer, and an apparatus for venting gas contained in a liquid. The dialyzer and apparatus for venting gas are located within the extracorporeal hemodialysis circuit so that blood flows from the patient, through the arterial tubing, through the dialyzer, through the apparatus for venting gas, and towards the venous tubing.

In another embodiment of this invention, an extracorporeal hemodialysis circuit includes arterial tubing for receiving unfiltered blood from a patient, venous tubing for providing filtered blood to a patient, a dialyzer, and an apparatus for controlling pressure differential. The dialyzer and apparatus for venting gas are located within the extracorporeal hemodialysis circuit so that blood flows from the patient, through the arterial tubing, through the dialyzer, through the apparatus for controlling pressure differential, and towards the venous tubing.

Embodiments described herein can provide advantages in operating a hemodialysis machine. By opening and closing a clamp to control the height of a gas-liquid interface in the gas collection chamber, liquid (such as blood or saline) can be prevented from contacting the semipermeable membrane positioned at the upper portion of the gas vent chamber. By reducing the pressure differential across the semipermeable membrane, the lifetime of the semipermeable membrane can be prolonged. These advantages result in decreased operator supervision, maintenance, and operating costs, as well as improved sterility.

### BRIEF DESCRIPTION OF THE DRAWINGS

FIG. 1 is a schematic diagram of a typical extracorporeal liquid circuit of a hemodialysis system.
FIG. 2 is a side view of a chamber for venting gas having two level detectors and a clamp.
FIG. 3 is a side view of a hemodialysis cassette for venting gas having two level detectors and a clamp.
FIG. 4 illustrates a control algorithm for venting gas in a system having two level detectors and a clamp.
FIG. 5 is a schematic illustrating the connectivity between a gas collection chamber, a pressure transducer, a gas pump, and a valve.
FIG. 6 illustrates a control algorithm for a first mode of operating an active gas venting system.
FIG. 7 illustrates a control algorithm for a second mode of operating an active gas venting system.
FIG. 8 illustrates a control algorithm for a third mode of operation of an active gas venting system.

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.

### DETAILED DESCRIPTION OF THE INVENTION

### Extracorporeal Circuit

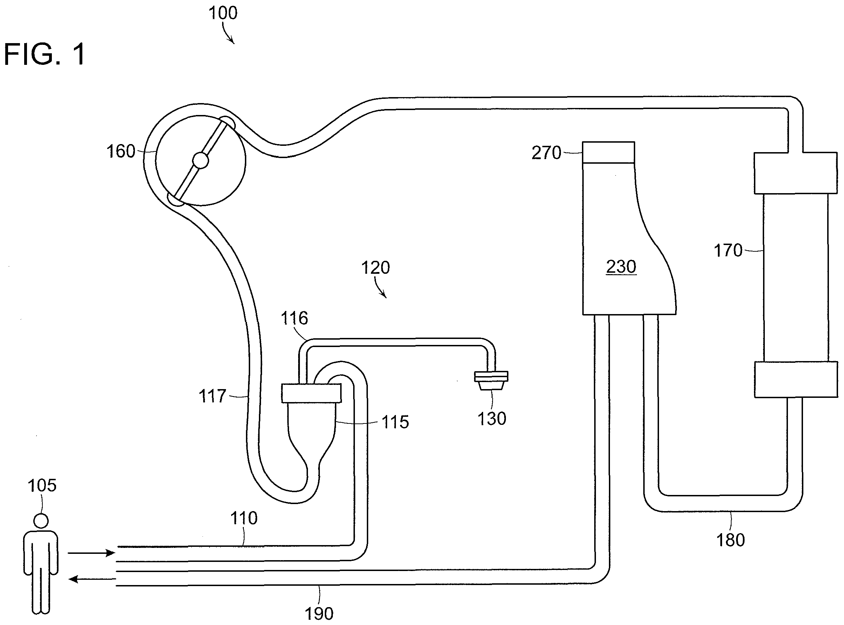

Figure 1 illustrates a typical extracorporeal hemodialysis circuit 100, which includes tubing through which the blood flows and components for filtering and performing hemodialysis on the blood. Blood flows from a patient 105 through arterial tubing 110. After exiting the patient, blood drips into a drip chamber 115 where a connecting tube 116 from the drip chamber 115 attaches to an arterial pressure sensor assembly 120 that determines the pressure of the blood on the arterial side of the circuit 100. The arterial pressure assembly 120 can include a pressure transducer 130 so that the pressure of blood flowing through the circuit 100 on the arterial side can be monitored.

A pump 160, such as a peristaltic pump, forces the blood to continue along the path through the circuit 100. After exiting the drip chamber 115, the blood then flows through tubing 117 to a dialyzer 170, which separates waste products and excess fluid from the blood. After passing through the dialyzer 170, the blood flows through venous tubing 180 towards a gas collection and venting chamber 230 in which gas (e.g., air) bubbles in the blood can separate from the blood and escape by passing across a semipermeable membrane 270 before the blood continues to the patient 105. As used herein, the term "semipermeable membrane" refers a membrane that is permeable to gas, but impermeable to liquid. Thus, the semipermeable membrane operates to inhibit liquids within the chamber 230 from escaping while allowing gas to escape from the chamber 230. After leaving the chamber 230, the blood travels through a venous line 190 and back to the patient 105. The gas collection apparatus and cassette subsequently described herein can be used with an extracorporeal hemodialysis circuit and device, as illustrated in Figure 1, or with other blood dialysis systems.

### Gas Collection Apparatus and Cassette

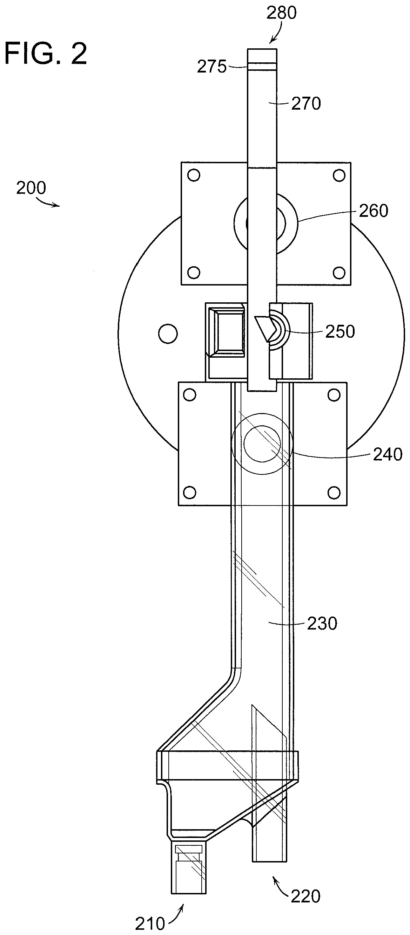

Figure 2 illustrates an exemplary embodiment of a gas venting apparatus 200 having a chamber, two level detectors, and a clamp. The gas venting apparatus 200 has a liquid inlet 210 and a liquid outlet 220. In Figure 2, the liquid inlet 210 is positioned below the liquid outlet 220, but the liquid inlet 210 can also be positioned above the liquid outlet 220 or at approximately the same height as the liquid outlet 220. A liquid, such as blood, enters through the liquid inlet 210 and leaves through the liquid outlet 220. The liquid enters the volume of the gas collection chamber 230 when the clamp 250 is in an open position.

The lower level detector 240 and the upper level detector 260 can detect the presence of a gas or a liquid. The clamp 250 can open or close based on signals from the lower level detector 240 and the upper level detector 260. During operation, the gas collection chamber initially fills with a liquid, such as blood, if the clamp 250 is in an open position. Upon detecting the presence of liquid at the upper level detector 260, the clamp 250 can close. The liquid can contain gas bubbles. Over time, the gas bubbles rise to the surface and begin to fill the gas collection chamber with the gas, thereby creating an interface between the gas and the liquid. As gas bubbles continue to rise to the surface, the interface between the gas and the liquid moves vertically down the gas collection chamber.

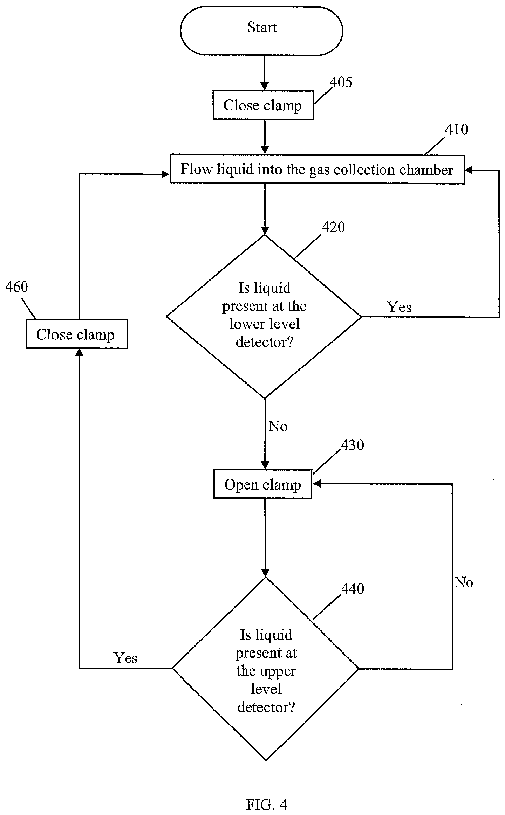

When the lower level detector 240 detects the presence of a liquid, the clamp 250 remains closed. When the gas-liquid interface crosses the location where the lower level detector is positioned, the level detector can send a signal indicative of the presence of a gas. The signal can be sent from the lower level detector 240 to a control apparatus, which is described with respect to Figure 4, that receives the signal. Upon receiving the signal, the control apparatus can send a signal to the clamp 250 instructing the clamp to open.

Once the clamp 250 opens, the gas in the gas collection chamber 230 can travel through the gas venting chamber 270. The gas venting chamber 270 has a semipermeable membrane 275 positioned at the upper portion and a gas outlet 280. In some embodiments, the outlet can vent gas to the atmosphere. When the clamp 250 is open, the gas collection chamber 230 is in fluid communication with the gas vent chamber 270, which in turn is in fluid communication with the atmosphere.

Ordinarily, the pressure in the gas collection chamber is greater than atmospheric pressure. Thus when the clamp 250 is open, the gas-liquid interface moves vertically up the gas collection chamber, which releases accumulated gas to the atmosphere.

Similar to the lower level detector, the upper level detector 260 detects the presence of a gas or a liquid. When the clamp 250 is open, the gas-liquid interface can move vertically up the chamber and can cross the location where the upper level detector 260 is positioned. When the upper level detector 260 detects the presence of a gas, the clamp 250 can remain open, thus permitting further venting of gas to the atmosphere. When the upper level detector 260 detects the presence of a liquid, the clamp 250 can close, thus preventing the liquid from reaching the gas outlet 280. In some embodiments, the upper level detector can send a signal indicative of the presence of a gas or a liquid to the control apparatus, which is described with respect to Figure 4. The control apparatus can then send a signal to the clamp that opens or closes the clamp.

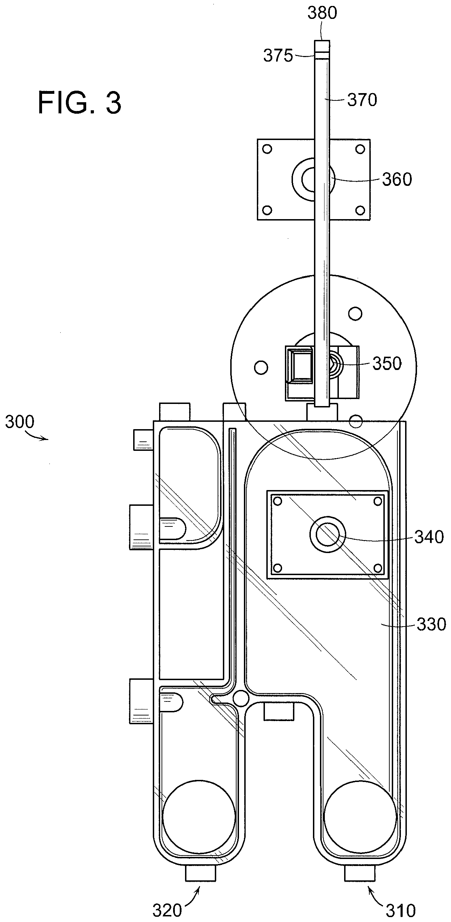

Figure 3 illustrates a gas venting cassette 300 having a chamber, two level detectors, and a clamp. The apparatus of Figure 3 is similar to that of Figure 2, except the apparatus of Figure 3 is a cassette 300 including a liquid inlet 310, a liquid outlet 320, a gas collection chamber 330, a lower level detector 340, a clamp 350, an upper level detector 360, a gas vent chamber 370, a semipermeable membrane 375, and a gas outlet 380. The embodiment of Figure 3 operates similarly to the embodiment of Figure 2. The primary difference between the embodiments of Figure 2 and Figure 3 is in the shape of the gas collection chamber.

The term "clamp" is used in its broadest sense, meaning that it is an element that is capable of opening and closing the gas vent chamber. In one embodiment, the clamps (250 and 350) can be pinch clamps that, in a closed position, exert pressure on a tube to prevent the passage of gas or liquid. In another embodiment, the clamps (250 and 350) can be balloon clamps. A wide variety of suitable devices that can open and close the gas vent chamber in response to a signal can be used. The clamp can completely close and completely open the gas vent chamber, or the clamp can partially close and partially open the gas vent chamber.

In one embodiment, the level detectors (240, 260, 340, and 360) can detect the density of a fluid. For example, the level detectors can be ultrasonic level detectors. Liquids have a higher density than gasses. Thus, the level detector can send a signal indicative of the density of a fluid, wherein the density is indicative of the presence of a gas or a liquid. In some embodiments, the liquid can be blood. In some embodiments, the gas can be air.

The gas bubbles can be dissolved in the liquid, or the gas bubbles can be too large to be considered dissolved in the liquid. In some cases, the gas bubbles can be observable with the naked eye. In other cases, the gas bubbles can on the order of magnitude of a millimeter or less.

The gas collection chamber (230 and 330) and the gas vent chamber (270 and 370) are not necessarily separate pieces. Rather, the two can be an integrated component. In other words, the gas collection chamber and the gas vent chamber can be a single, integral unit. While Figues 2 and 3 illustrate the lower level detector (240 and 340) positioned at the gas collection chamber (230 and 330) and the upper level detector (260 and 360) positioned at the gas vent chamber (270 and 370), the lower and upper level detectors (240, 260, 340, and 360) can be positioned at either the gas collection chamber (230 and 330) or at the gas vent chamber (270 and 370).

Typically, the gas outlet (280 and 380) is capped with a semipermeable membrane (275 and 375) made from polymers such as a polytetrafluoroethylene (PTFE) or polyethylene (PE), though other suitable semipermeable membranes can also be used. Typically, the semipermeable membrane is a hydrophobic membrane. Typically, the semipermeable membrane is a microporous membrane. Preferably, the semipermeable has a pore size between 0.1 micron to 0.22 microns. Suitable membranes are manufactured by W. L. Gore & Associates, Inc., Millipore Corporation, and Pall Corporation.

The chamber embodiment of Figure 2 and the cassette embodiment of Figure 3 can be made of a wide variety of biocompatible materials suitable for medical applications, and can be formed into the appropriate shape by any processes suitable for medial applications. For example, the cassette can typically be formed from polymers such as polyvinyl chloride (PVC) and polycarbonate (PC).

Typically, the liquid inlet (210 and 310) and the liquid outlet (220 and 320) connect to tubing. As shown in Figure 1, the liquid inlet (210 and 310) connects to tubing 180, and the liquid outlet (220 and 320) connect to tubing 190. The tubing can be of a wide variety of biocompatible materials suitable for medical application.

### Operation of the Gas Collection Apparatus and Cassette

Figure 4 is a flowchart illustrating steps in a method for venting gas in a system having a gas collection chamber, two level detectors, and a clamp. In one embodiment, the clamp is initially in a closed position. Optionally, the clamp can be closed if it is open (step 405). Liquid flows into the gas collection chamber (step 410). The liquid enters the gas collection chamber via a liquid inlet, such as liquid inlet 210 or 310. The lower level detector detects the presence of a liquid or gas (step 420). If liquid is present at the lower level detector (i.e., if gas is not present), the clamp remains closed and liquid continues to flow into the gas collection chamber (step 410). If liquid is not present at the lower level detector (i.e., if gas is present at the lower level detector), the clamp opens (step 430).

While the clamp is open, gas in the gas collection chamber is in fluid communication with the atmosphere via a gas venting chamber, and gas can vent to the atmosphere. As gas vents to the atmosphere, the gas-liquid interface rises, and the upper level detector detects the presence of a liquid or a gas (step 440). If liquid is not present at the upper level detector (i.e., if gas is present), the clamp remains open (step 430), and the upper level detector continues to detect the presence of liquid or gas (step 440). If liquid is present at the upper level detector (i.e., if gas is not present), then the clamp closes (step 460). Liquid continues to flow into the gas collection chamber (step 410) and the cycle repeats.

While Figures 4 describes detecting whether liquid is present at the upper level detector at step 440, the upper level detector can detect the presence of liquid or gas at the outset. However, the system and method do not need to consider the presence or absence of gas or liquid at the upper level detector until the clamp has opened.

While steps 420 and 440 describe detecting whether liquid is present at the level detectors, it is equivalent to detect whether gas is present at the level detectors. In such case, the relative placement of the "Yes" and "No" answers to the inquiry are reversed. In other words, if gas is present at the lower level detector (step 420), then the clamp opens (step 430), and if gas is not present, then the clamp remains closed (step 410). Similarly, if gas is present at the upper level detector (step 440), then the clamp remains open (step 430), and if gas is not present, then the clamp closes (step 460).

### Apparatus for Controlling Pressure Differential

Another embodiment is an apparatus and method for controlling the pressure differential across a semipermeable membrane by providing backpressure to the semipermeable membrane (*i.e.,* in the direction opposite to the direction applied by the flow of gas exiting the system). Thus, the present apparatus and method can prolong the lifetime of the semipermeable membrane as well as provide a means to actively control the level of liquid, such as blood and/or saline, in the gas venting chamber.

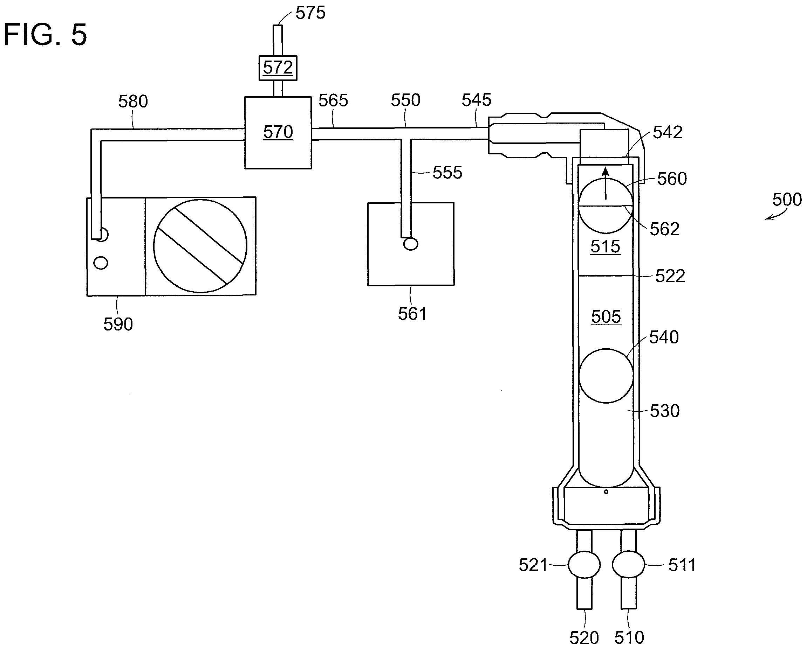

Figure 5 is a schematic illustration of the connectivity of the active venting apparatus and method 500. The gas collection chamber 530 collects gas that has been released from liquid entering the gas collection chamber 530 via inlet 510 and exiting via outlet 520. Pressure sensors 511 and 521 can monitor the inlet and outlet pressures. In a typical hemodialysis setup, the liquid is blood 505 and saline 515 that meet at an interface 522. The gas collection chamber 530 can also have a lower level detector 540 and an upper level detector 560, as described above. As illustrated in Figure 5, the upper level detector 560 is positioned to sense gas or liquid near the top of the gas collection chamber 530. As previously described, the level detectors (540 and 560) can detect the density of a fluid. For example, the level detectors can be ultrasonic level detectors.

Near the top of the gas collection chamber is a semipermeable membrane 542 that serves as a barrier between the hemodialysis circuit, such as the extracorporeal hemodialysis circuit 100 of Figure 1, and the environment. The semipermeable membrane 542 can be a hydrophobic membrane. When the valve 572 is open, gas passes across the semipermeable membrane 542 and along the exit pathway 545 toward branch point 550. Branch 555 extends to a pressure sensor 561 that monitors the pressure along the exit pathway 545. The pressure sensor 561 can be referred to as the machine-side pressure sensor to distinguish it from the bloodline pressure sensors (e.g., 511 and 521). The machine-side pressure sensor 561 ordinarily does not directly contact biohazardous liquids. The exit pathway extends along pathway 565 into the T-connector 570, which extends to the atmosphere 575 via valve 572 and to a gas pump 590 via pathway 580. Alternatively, a source of pressurized gas, such as pressurized air or nitrogen, can be provided instead of a gas pump 590.

During operation of a hemodialysis machine, the gas collection chamber 530 becomes increasingly filled with gas, and the gas-liquid interface 562 moves downward. Gas exits the gas collection chamber 530 by flowing across the semipermeable membrane 542 and along the exit pathway 545 and 565 into the T-connector 570. The gas vents to the atmosphere 575 via valve 572.

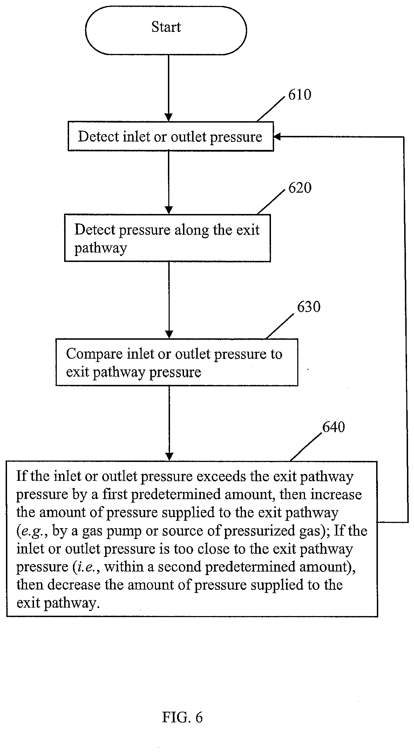

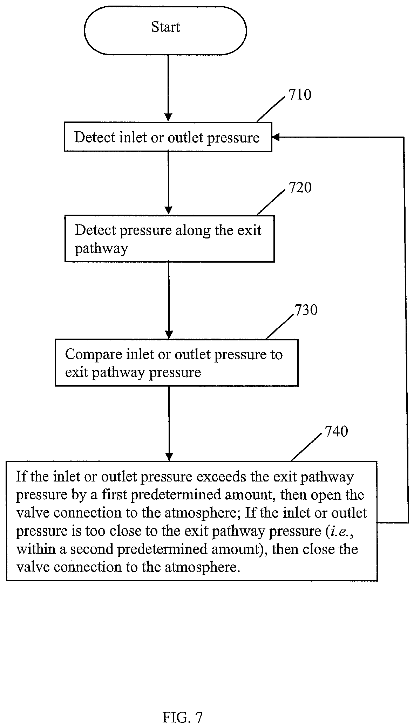

In a first and second mode of operation, the machine side pressure sensor 561 and the inlet pressure sensor 511 can be operably connected to a control apparatus, which is described with respect to Figures 6 and 7. The control apparatus can compare the inlet pressure as measured by the inlet pressure sensor 511 to the pressure as measured by the machine-side pressure sensor 561. The pressure along the exit pathway 545 can be adjusted by controlling the gas source 590 to increase or decrease the pressure. The pressure along the exit pathway 545 can also be adjusted by opening or closing valve 572. Thus, the amount of pressure applied to the backside of the semipermeable membrane can be adjusted so that it is only slightly less than the pressure applied on the gas vent side. Alternatively, the outlet pressure sensor 521 can be operably connected to a control apparatus, and the control apparatus can compare the outlet pressure as measured by the outlet pressure sensor 521 to the pressure as measured by the machine-side pressure sensor 561. In addition, both the inlet and outlet pressures can be measured and compared to the machine-side pressure sensor 561. Typical venous or arterial pressures are 100 mmHg to 500 mmHg, or more typically 100 mmHg to 200 mmHg.

Figure 6 illustrates a control algorithm for a first mode of operating an active venting system, such as that illustrated in Figure 5. The inlet pressure and the pressure along the exit pathway are detected (steps 610 and 620). Alternatively, the outlet pressure can be detected rather than the inlet pressure, or the outlet pressure can be detected in addition to the inlet pressure. The sequence of steps 610 and 620 can be reversed, such that the pressure along the exit pathway is detected before the inlet pressure is detected. Alternatively, steps 610 and 620 can be combined into a single step that is performed simultaneously. Next, the control apparatus compares the inlet or outlet pressure to the exit pathway pressure (step 630). If the inlet or outlet pressure exceeds the exit pathway pressure by a first predetermined amount, then the amount of pressure supplied to the exit pathway (*e.g.,* by a gas pump or source of pressurized gas) is increased. If the inlet or outlet pressure is too close to the exit pathway pressure (*i.e.,* within a second predetermined amount), then the amount of pressure supplied to the exit pathway is decreased (step 640). The first and second predetermined amounts can be selected by an operator of a hemodialysis machine.

Figure 7 illustrates a control algorithm for a second mode of operating an active venting system, such as that illustrated in Figure 5. The inlet pressure and the pressure along the exit pathway are detected (steps 710 and 720). Alternatively, the outlet pressure can be detected rather than the inlet pressure, or the outlet pressure can be detected in addition to the inlet pressure. The sequence of steps 710 and 720 can be reversed, such that the pressure along the exit pathway is detected before the inlet pressure is detected. Alternatively, steps 710 and 720 can be combined into a single step that is performed simultaneously. Next, the control apparatus compares the inlet or outlet pressure to the exit pathway pressure (step 730). If the inlet or outlet pressure exceeds the exit pathway pressure by a first predetermined amount, then the valve 572 can open the connection to the atmosphere 575. If the inlet or outlet pressure is too close to the exit pathway pressure (*i.e.,* within a second predetermined amount), then the valve 572 can close the connection to the atmosphere 575 (step 640). The first and second predetermined amounts can be selected by an operator of a hemodialysis machine. The valve 572 that opens and closes the connection to the atmosphere 575 need not be fully closed or fully opened, but can be partially opened or partially closed relative to the difference between the inlet pressure exceeds the exit pathway pressure.

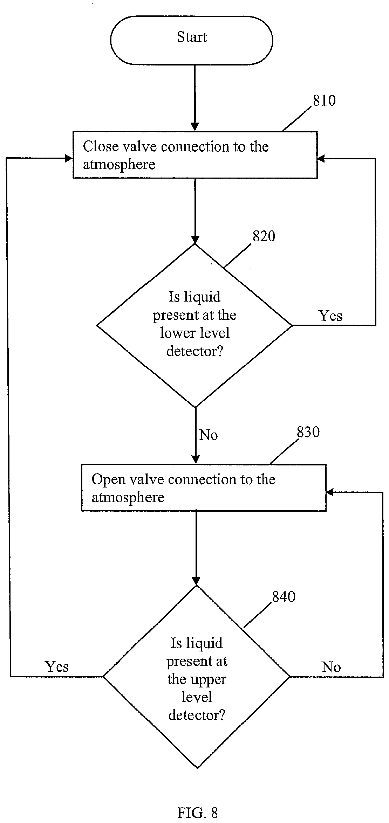

In a third mode of operation, the lower level detector 540 and upper level detector 560 can detect the presence of a liquid or a gas, as described above. When the lower level detector detects the presence of a gas, a signal can be sent to a control apparatus, which is described with respect to Figure 8. The control apparatus can send a signal to open and close the valve 572 controlling the connection to the atmosphere 575. For example, the control apparatus can open the valve 572 when the lower level detector 540 detects gas and closes the valve 572 when the upper level detector 560 detects liquid.

Figure 8 illustrates a control algorithm for a third mode of operating an active venting system, such as that illustrated in Figure 5. At the outset, the valve connection to the atmosphere is closed (step 810). If liquid is present at the lower level detector, the valve remains closed (step 820). If liquid is not present at the lower level detector, the valve connection to the atmosphere opens (step 830). Then, if liquid is present at the upper level detector (step 840), the valve is closed (step 810). If liquid is not present at the upper level detector (step 840), then the valve connection to the atmosphere remains open (step 830).

The apparatus for controlling pressure differential can serve to reduce the pressure differential across the semipermeable membrane 542, which can reduce the damage to the membrane caused by excessive pressure differential across the membrane. In the inventors' experience, the semipermeable membrane can be fragile and can tend to leak liquid, particularly over the course of an operation of extended duration (e.g., 24 to 72 hours). By reducing or preventing the incidence of rips, tears, breaks, or other leaks in the membrane 542, the operational lifetime of the membrane can be increased, thereby reducing operating costs and improving ease of operation in a clinical or home setting. The increased reliability provided by the active venting system and method can reduce the amount of operator intervention required during a typical hemodialysis procedure.

While this invention has been particularly shown and described with references to example embodiments thereof, it will be understood by those skilled in the art that various changes in form and details may be made therein without departing from the scope of the invention encompassed by the appended claims.

## Claims

1. An extracorporeal hemodialysis circuit comprising:
a) arterial tubing (110) for receiving unfiltered blood from a patient;
b) venous line (190) for providing filtered blood to the patient; and
c) a dialyzer and an apparatus for controlling pressure differential located within the extracorporeal hemodialysis circuit so that blood flows from the patient, through the arterial tubing, through the dialyzer, through the apparatus for controlling differential pressure, and into the venous line, wherein the apparatus for controlling pressure differential controls pressure differential across a semipermeable membrane sealing an exit pathway from a gas collection chamber (530), the apparatus for controlling pressure differential comprising:
i) a first pressure sensor (561) that senses the pressure along the exit pathway from the gas collection chamber;
ii) a second pressure sensor (521, 511) that senses an inlet or outlet pressure of the gas collection chamber;
iii) a semipermeable membrane (542) disposed between the gas collection chamber and the exit pathway;
iv) a valve (572) operably connected to a gas source, the exit pathway from the gas collection chamber, and the atmosphere; and
v) a control apparatus connected to the first pressure sensor and the valve;
wherein the control apparatus can vary the pressure provided by the pressurized gas source in order to vary the amount of backpressure applied across the semipermeable membrane.

2. The extracorporeal hemodialysis circuit of claim 1, wherein a gas pump provides the gas source.

3. The extracorporeal hemodialysis circuit of claim 1, wherein a pressurized gas provides the gas source.

4. The extracorporeal hemodialysis circuit of claim 3, wherein the pressurized gas is air.

5. The extracorporeal hemodialysis circuit of claim 3, wherein the pressurized gas is nitrogen.

6. The extracorporeal hemodialysis circuit of claim 1, wherein the control apparatus compares the pressure measured by the first pressure sensor and the second pressure sensor and controls the gas source so that the amount of pressure along the exit pathway is less than the pressure measured by the second pressure sensor.

7. The extracorporeal hemodialysis circuit of claim 6, wherein the pressure sensed by the second pressure sensor is approximately 100 mmHg to 200 mmHg.

8. The extracorporeal hemodialysis circuit of claim 1, further including a lower level detector located in the gas collection chamber, and an upper level detector located in the gas collection chamber, wherein the lower level detector is located below the upper level detector, and wherein the lower and upper level detectors are capable of detecting gas or liquid.

9. The extracorporeal hemodialysis circuit of claim 8, wherein the control apparatus opens the valve connection to the atmosphere when the lower level detector detects gas and closes the valve connection to the atmosphere when the upper level detector detects liquid.

10. An apparatus for controlling the pressure differential across a semipermeable membrane sealing an exit pathway from a gas collection chamber that receives liquid-releasing gas, comprising:
a) a gas source for applying pressure to the exit pathway side of the semipermeable membrane;
b) a valve operably connected to the gas source, the exit pathway, and the atmosphere;
c) a first pressure sensor that senses the pressure along the exit pathway side;
d) a second pressure sensor that detects an inlet or outlet pressure of the gas collection chamber; and
e) control means connected to the first and second pressure sensors and the valve;
wherein the control means can vary the gas source in order to vary the amount of backpressure applied across the semipermeable membrane.

## Patentansprüche

1. Extrakorporaler Hämodialyse-Kreislauf, Folgendes umfassend:
a) einen Arterienschlauch (110) zur Aufnahme ungefilterten Bluts von einem Patienten,
b) eine Venenleitung (190) zum Bereitstellen gefilterten Bluts für den Patienten und
c) einen Dialysator und eine Vorrichtung zum Steuern eines Druckunterschieds, die derart in dem extrakorporalen Hämodialyse-Kreislauf angeordnet sind, dass Blut vom Patienten durch den Arterienschlauch, durch den Dialysator, durch die Vorrichtung zum Steuern eines Druckunterschieds und in die Venenleitung fließt, wobei die Vorrichtung zum Steuern eines Druckunterschieds den Druckunterschied über eine halbdurchlässige Membran steuert, die einen Austrittsweg von einer Gassammelkammer (530) verschließt, wobei die Vorrichtung zum Steuern des Druckunterschieds Folgendes umfasst:
I) einen ersten Drucksensor (561), der den Druck entlang des Austrittsweges von der Gassammelkammer erfasst,
II) einen zweiten Drucksensor (521, 511), der einen Ein- oder Auslassdruck der Gassammelkammer erfasst,
III) eine halbdurchlässige Membran (542), die zwischen der Gassammelkammer und dem Austrittsweg angeordnet ist,
IV) ein Ventil (572), das funktionsfähig mit einer Gasquelle, dem Austrittsweg aus der Gassammelkammer und der Umgebung verbunden ist, und
V) eine Steuervorrichtung, die mit dem ersten Drucksensor und dem Ventil verbunden ist,
wobei die Steuervorrichtung den Druck verändern kann, der durch die mit Druck beaufschlagte Gasquelle bereitgestellt wird, um den Umfang des Rückdrucks zu verändern, der an der halbdurchlässigen Membran anliegt.

2. Extrakorporaler Hämodialyse-Kreislauf nach Anspruch 1, wobei eine Gaspumpe die Gasquelle bereitstellt.

3. Extrakorporaler Hämodialyse-Kreislauf nach Anspruch 1, wobei ein mit Druck beaufschlagtes Gas die Gasquelle bereitstellt.

4. Extrakorporaler Hämodialyse-Kreislauf nach Anspruch 3, wobei das mit Druck beaufschlagte Gas Luft ist.

5. Extrakorporaler Hämodialyse-Kreislauf nach Anspruch 3, wobei das mit Druck beaufschlagte Gas Stickstoff ist.

6. Extrakorporaler Hämodialyse-Kreislauf nach Anspruch 1, wobei die Steuervorrichtung die Drücke vergleicht, die durch den ersten Drucksensor und den zweiten Drucksensor gemessen werden, und die Gasquelle derart steuert, dass die Höhe des Drucks entlang des Austrittsweges geringer als der Druck ist, der durch den zweiten Drucksensor gemessen wird.

7. Extrakorporaler Hämodialyse-Kreislauf nach Anspruch 6, wobei der vom zweiten Drucksensor erfasste Druck ungefähr 100 mmHg bis 200 mmHg beträgt.

8. Extrakorporaler Hämodialyse-Kreislauf nach Anspruch 1, ferner einen Detektor für den unteren Pegel beinhaltend, der in der Gassammelkammer angeordnet ist, und einen Detektor für den oberen Pegel, der in der Gassammelkammer angeordnet ist, wobei der Detektor für den unteren Pegel unter dem Detektor für den oberen Pegel angeordnet ist und wobei die Detektoren für den unteren und den oberen Pegel in der Lage sind, Gas oder Flüssigkeit zu erkennen.

9. Extrakorporaler Hämodialyse-Kreislauf nach Anspruch 8, wobei die Steuervorrichtung die Ventilverbindung zur Umgebung öffnet, wenn der Detektor für den unteren Pegel Gas erkennt, und die Ventilverbindung zur Umgebung schließt, wenn der Detektor für den oberen Pegel Flüssigkeit erkennt.

10. Vorrichtung zum Steuern des Druckunterschieds an einer halbdurchlässigen Membran, die einen Austrittsweg von einer Gassammelkammer verschließt, die Flüssigkeit freisetzendes Gas aufnimmt, Folgendes umfassend:
a) eine Gasquelle zum Anlegen von Druck auf die Seite des Austrittsweges der halbdurchlässigen Membran,
b) ein Ventil, das funktionsfähig mit der Gasquelle, dem Austrittsweg und der Umgebung verbunden ist,
c) einen ersten Drucksensor, der den Druck entlang der Seite des Austrittsweges erfasst,
d) einen zweiten Drucksensor, der einen Ein- oder Auslassdruck der Gassammelkammer erkennt, und
e) Steuermittel, die mit dem ersten und dem zweiten Drucksensor und dem Ventil verbunden sind,
wobei die Steuermittel den Gasdruck verändern können, um den Umfang des Rückdrucks zu verändern, der an der halbdurchlässigen Membran anliegt.

## Revendications

1. Circuit d'hémodialyse extracorporel comprenant :
(a) une tubulure artérielle (110) pour recevoir du sang non filtré d'un patient ;
(b) un tube de veine (190) pour fournir du sang filtré au patient ; et
(c) un dialyseur et un appareil pour commander un différentiel de pression situé dans le circuit d'hémodialyse extracorporel de sorte que le sang s'écoule du patient, à travers la tubulure artérielle, à travers le dialyseur, à travers l'appareil pour commander la pression différentielle, et dans le tube de veine, dans lequel l'appareil pour commander le différentiel de pression commande le différentiel de pression de part et d'autre d'une membrane semi-perméable scellant un trajet de sortie d'une chambre de collecte de gaz (530), l'appareil pour commander le différentiel de pression comprenant :
(i) un premier capteur de pression (561) qui détecte la pression le long du trajet de sortie de la chambre de collecte de gaz ;
(ii) un deuxième capteur de pression (521, 511) qui détecte une pression d'entrée ou de sortie de la chambre de collecte de gaz ;
(iii) une membrane semi-perméable (542) disposée entre la chambre de collecte de gaz et le trajet de sortie ;
(iv) une vanne (572) reliée de manière fonctionnelle à une source de gaz, au trajet de sortie de la chambre de collecte de gaz et à l'atmosphère ; et
(v) un appareil de commande connecté au premier capteur de pression et à la vanne ;
dans lequel l'appareil de commande peut modifier la pression fournie par la source de gaz sous pression afin de modifier la quantité de contre-pression appliquée de part et d'autre de la membrane semi-perméable.

2. Circuit d'hémodialyse extracorporel selon la revendication 1, dans lequel une pompe à gaz réalise la source de gaz.

3. Circuit d'hémodialyse extracorporel selon la revendication 1, dans lequel un gaz sous pression réalise la source de gaz.

4. Circuit d'hémodialyse extracorporel selon la revendication 3, dans lequel le gaz sous pression est de l'air.

5. Circuit d'hémodialyse extracorporel selon la revendication 3, dans lequel le gaz sous pression est de l'azote.

6. Circuit d'hémodialyse extracorporel selon la revendication 1, dans lequel l'appareil de commande compare la pression mesurée par le premier capteur de pression et le deuxième capteur de pression et commande la source de gaz de sorte que la quantité de pression le long du trajet de sortie soit inférieure à la pression mesurée par le deuxième capteur de pression.

7. Circuit d'hémodialyse extracorporel selon la revendication 6, dans lequel la pression détectée par le deuxième capteur de pression est d'environ 100 mmHg à 200 mmHg.

8. Circuit d'hémodialyse extracorporel selon la revendication 1, comprenant en outre un détecteur de niveau inférieur situé dans la chambre de collecte de gaz, et un détecteur de niveau supérieur situé dans la chambre de collecte de gaz, dans lequel le détecteur de niveau inférieur est situé au-dessous du détecteur de niveau supérieur, et dans lequel les détecteurs de niveaux inférieur et supérieur sont capables de détecter un gaz ou un liquide.

9. Circuit d'hémodialyse extracorporel selon la revendication 8, dans lequel l'appareil de commande ouvre la liaison de vanne vers l'atmosphère lorsque le détecteur de niveau inférieur détecte un gaz et ferme la liaison de vanne vers l'atmosphère lorsque le détecteur de niveau supérieur détecte un liquide.

10. Appareil pour commander le différentiel de pression de part et d'autre d'une membrane semi-perméable scellant un trajet de sortie d'une chambre de collecte de gaz qui reçoit un gaz de libération de liquide, comprenant :
(a) une source de gaz pour appliquer une pression au côté de trajet de sortie de la membrane semi-perméable ;
(b) une vanne reliée de manière fonctionnelle à la source de gaz, au trajet de sortie et à l'atmosphère ;
(c) un premier capteur de pression qui détecte la pression le long du côté de trajet de sortie ;
(d) un deuxième capteur de pression qui détecte une pression d'entrée ou de sortie de la chambre de collecte de gaz ; et
(e) des moyens de commande connectés aux premier et deuxième capteurs de pression et à la vanne ;
dans lequel les moyens de commande peuvent modifier la source de gaz afin de modifier la quantité de contre-pression appliquée de part et d'autre de la membrane semi-perméable.
